(19) Europäisches Patentamt
European Patent Office
Office européen des brevets

(11) EP 0 709 358 B1

(12) **EUROPEAN PATENT SPECIFICATION**

(45) Date of publication and mention of the grant of the patent:
**13.10.1999 Bulletin 1999/41**

(21) Application number: **94918548.2**

(22) Date of filing: **22.06.1994**

(51) Int. Cl.$^6$: **C07C 39/15**, C07C 43/23, C07D 275/03, A01N 43/80

(86) International application number:
**PCT/JP94/00997**

(87) International publication number:
**WO 95/00465 (05.01.1995 Gazette 1995/02)**

# (54) NOVEL CLATHRATE COMPOUND, PROCESS FOR PRODUCING THE SAME, AND ANTIFOULING AGENT

NEUE CLATHRAT-VERBINDUNGEN, VERFAHREN ZU IHRER HERSTELLUNG UND FÄULMISVERHINDERNDES MITTEL

NOUVEAU COMPOSE CLATHRATE, PROCEDE POUR SA PRODUCTION, ET AGENT ANTISALISSURE

(84) Designated Contracting States:
**DE FR GB**

(30) Priority: **23.06.1993 JP 17601893**

(43) Date of publication of application:
**01.05.1996 Bulletin 1996/18**

(73) Proprietor:
**NIPPON SODA CO., LTD.**
**Chiyoda-ku, Tokyo 100-8165 (JP)**

(72) Inventors:
- **SUZUKI, Hiroshi**
  **Chiba 299-01 (JP)**
- **ICHIKAWA, Takako**
  **Chiba-shi Chiba 262 (JP)**
- **YANAGI, Yoshikazu**
  **Setagaya-ku Tokyo 158 (JP)**

(74) Representative:
**de Bruijn, Leendert C. et al**
**Nederlandsch Octrooibureau**
**P.O. Box 29720**
**2502 LS Den Haag (NL)**

(56) References cited:
**WO-A-93/12060**
**JP-A- 3 279 373**
**JP-A- 4 066 541**
**JP-A- 5 004 978**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7/3.6

## Description

The present invention relates to a new clathrate compound, and particularly relates to a clathrate compound comprising tetrakisphenol as the host compound and 4,5-dichloro-2-alkyl-3(2H) isothiazolon as the guest compound, its preparation method and a stain-proofing agent containing such compound.

## Background of the Invention:

4,5-dichloro-2-alkyl-3(2H) isothiazolons including 4,5-dichloro-2-n-octyl-3(2H) isothiazolon are commonly used as industrial fungicides for slime control, stain-proofing agents and other applications. They have a drawback of harsh effect to human bodies during application including high skin irritation and tendency to cause rash.

It is an object of the present invention to provide a new clathrate compound having 4,5-dichloro-2-alkyl-3(2H) isothiazolons as the guest compound and its preparation procedures as a safer 4,5-dichloro-2-alkyl-3(2H) isothiazolon.

## Disclosure of the Invention:

Having devoted themselves to their study to attain the above object, inventors successfully achieved the present invention with finding that, by directly adding a certain tetrakisphenol host compound to the liquid containing 4,5-dichloro-2-alkyl-3(2H) isothiazolon for reaction or by mixing tetrakisphenol host compound powder and 4,5-dichloro-2-alkyl-3(2H) isothiazolon powder for direct solid phase reaction, new clathrate compound can be quite effectively generated and that it is effective as a stain-proofing agent.

In other words, the present invention relates to such a new clathrate compound, its preparation method and a stain-proof agent characterized by that it contains one or a plurality of such clathrates as effective constituent.

Given below is the detailed description of the present invention.

The tetrakisphenol used as the host compound in the present invention may be the compound expressed by the formula (I) below.

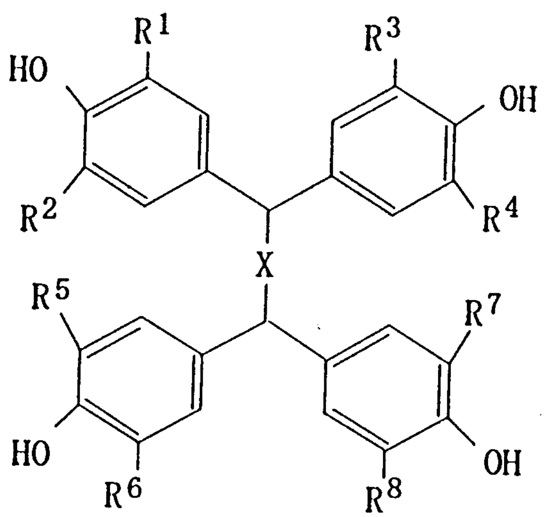

(I)

(In the formula, X represents $(CH_2)n$ where n may be 0,1, 2 or 3; and each of R1 to R8 represents one component selected from the group consisting of hydrogen atom, lower-alkyl group, halogen atom, lower-alkoxy group and substitutable phenyl group).

In the above, lower-alkyl group is a methyl, ethyl, propyl or isopropyl group, and lower-alkoxy group is a methoxy, ethoxy, propoxy or isopropoxy group.

Specific examples of tetrakisphenols include 1,1,2,2-tetrakis (4-hydroxyphenyl) ethane, 1,1,2,2-tetrakis (3-methyl-4-hydroxyphenyl) ethane, 1,1,2,2-tetrakis (3-bromo-4-hydroxypheny) ethane, 1,1,2,2-tetrakis (3,5-dimethyl-4-hydroxyphenyl) ethane, 1,1,2,2-tetrakis (3-t-butyl-4-hydroxyphenyl) ethane, 1,1,2,2-tetrakis (3-chloro-4-hydroxyphenyl)

ethane, 1,1,2,2-tetrakis (3,5-dichloro-4-hydroxyphenyl) ethane, 1,1,2,2-tetrakis (3-fluoro-4-hydroxyphenyl) ethane, 1,1,2,2-tetrakis (3,5-difluoro-4-hydroxyphenyl) ethane, 1,1,2,2-tetrakis (3-methoxy-4-hydroxyphenyl) ethane, 1,1,2,2-tetrakis (3,5-dimethoxy-4-hydroxyphenyl) ethane, 1,1,2,2-tetrakis (3-chloro-5-methyl -4-hydroxyphenyl) ethane, 1,1,2,2-tetrakis (3-chloro-5-phenyl-4-hydroxyphenyl) ethane, 1,1,2,2-tetrakis [(4-hydroxy-3-phenyl) phenyl] ethane, 1,1-bis (4-hydroxyphenyl)-2,2-bis(3-methyl-4-hydroxyphenyl) ethane, 1,1,3,3-tetrakis(4-hydroxyphenyl) propane, 1,1,3,3-tetrakis (3-methyl-4-hydroxyphenyl) propane, 1,1,3,3-tetrakis (3,5-dimethyl-4-hydroxyphenyl) propane, 1,1,3,3-tetrakis (3-chloro-4-hydroxyphenyl) propane, 1,1,3,3-tetrakis (3,5-dichloro-4-hydroxyphenyl) propane, 1,1,3,3-tetrakis (3-fluoro-4-hydroxyphenyl) propane, 1,1,3,3-tetrakis (3,5-difluoro-4-hydroxyphenyl) propane, 1,1,3,3-tetrakis (3-phenyl-4-hydroxyphenyl) propane, 1,1,4,4-tetrakis (4-hydroxyphenyl) butane, 1,1,4,4-tetrakis (3-methyl-4-hydroxyphenyl) butane, 1,1,4,4-tetrakis (3,5-dimethyl-4-hydroxyphenyl) butane, 1,1,4,4-tetrakis (3-chloro-4-hydroxyphenyl) butane, 1,1,4,4-tetrakis (3,5-dichloro-4-hydroxyphenyl) butane, 1,1,4,4-tetrakis (3-fluoro-4-hydroxyphenyl) butane and 1,1,4,4-tetrakis (3,5-difluoro-4-hydroxyphenyl) butane.

The alkyl group in 4,5-dichloro-2-alkyl-3(2H) isothiazolon, which serves as the guest compound in the present invention, is a normal-chain or branching-chain alkyl group having 1 to 20 carbons.

Specific examples of 4,5-dichloro-2-alkyl-3(2H) isothiazolon include 4,5-dichloro-2-methyl-3(2H) isothiazolon, 4,5-dichloro-2-ethyl-3(2H) isothiazolon, 4,5-dichloro-2-isopropyl-3(2H) isothiazolon, 4,5-dichloro-2-n-propyl-3(2H) isothiazolon, 4,5-dichloro-2-n-butyl-3(2H) isothiazolon, 4,5-dichloro-2-sec-butyl-3(2H) isothiazolon, 4,5-dichloro-2-t-butyl-3(2H) isothiazolon, 4,5-dichloro-2-n-pentyl-3(2H) isothiazolon, 4,5-dichloro-2-neopentyl-3(2H) isothiazolon, 4,5-dichloro-2-n-hexyl-3(2H) isothiazolon, 4,5-dichloro-2-c-hexyl-3(2H) isothiazolon, 4,5-dichloro-2-n-heptyl-3(2H) isothiazolon, 4,5-dichloro-2-n-octyl-3(2H) isothiazolon, 4,5-dichloro-2-n-nonyl-3(2H) isothiazolon, 4,5-dichloro-2-n-decyl-3(2H) isothiazolon, 4,5-dichloro-2-n-undecyl-3(2H) isothiazolon and 4,5-dichloro-2-n-dodecyl-3(2H) isothiazolon.

The new clathrate compound of the present invention can be obtained by mixing a solution where 4,5-dichloro-2-alkyl-3(2H) isothiazolon is solved into alcohol or other organic solvent and tetrakisphenol as the host compound and stirring them at a temperature from the room temperature to 100°C for a couple of minutes to a couple of hours for reaction so that 4,5-dichloro-2-alkyl-3(2H) isothiazolon is easily clathrated to the host compound.

Alternatively, by grinding and mixing 4,5-dichloro-2-alkyl-3(2H) isothiazolon and tetrakisphenol powder as the host compound at a temperature from the room temperature to 60°C for a couple of minutes to a couple of hours, solid phase reaction or solid-liquid reaction is caused and 4,5-dichloro-2-alkyl-3(2H) isothiazolon is clathrated into the host compound and the new clathrate compound of the present invention can be obtained.

The host compound of the present invention selectively conducts clathration of 4,5-dichloro-2-alkyl-3(2H) isothiazolon alone. This characteristic can be used for separation and collection of 4,5-dichloro-2-alkyl-3(2H) isothiazolon from organic solvent solution.

The clathrate compound obtained according to the present invention is free from irritative property against skin and eyes or irritating odor, which is drawback in handling of 4,5-dichloro-2-alkyl-3(2H) isothiazolons including 4,5-dichloro-2-n-octyl-3(2H) isothiazolon. It is thus easily handled and can be utilized as stain-proofing agents.

Stain-proofing agent applications include slime control agents, stain-proofing and mildew-proofing paints and stain-proofing agents for fishing nets.

An example of stain-proofing and mildew-proofing paints is the ship bottom stain-proofing paint.

Stain-proofing and mildew-proofing paints are obtained by mixing and dissolving or dispersing the following with at least one compound of the clathrate compounds, resins such as natural resin, oil resin, synthetic resin, synthetic rubber for general paints, organic solvent where these resins are to be dissolved, if required coloring agent, (soluble) various additives such as plasticizer, etc.

Preferable resins are various kinds of acrylic resin, polybutene, rosin, cumarone, vinyl chloride, chlorinated rubber, various kinds of alkyd, ethylene-vinyl acetate copolymerized resin, etc. The amount of the clathrate compound to be used is 0.1 to 50% of the paint. 0.1% to 50% for stain-proofing component of copper suboxide, etc. other than the clathrate compound can be added.

The fishing net stain-proofing agent consists of resin constituents such as acrylic resin, chlorinated rubber and rosin, solvents such as xylene, methylnaphthylene and pseudocumene, and the clathrate compound. Further, elusion adjusting agent, thickener, plasticizer or decomposition prevention agent may be added as additives if required. Preferable amounts to be used are 0.1 to 30% for effective stain-proofing component, 10 to 30% for resins, 60 to 80% for solvents and 0 to 5% for other additives. In addition, 0.1 to 50% for stain-proofing component such as cuprous oxide, etc. other than the clathrate compound can be added.

**Brief Description of the Drawings:**

Fig. 1 is a diagram to show the IR spectrum of the clathrate compound obtained in Embodiments 1,2 and 3

(host:guest = 1:1);

Fig. 2 is a diagram to show the IR spectrum of the clathrate compound obtained in Embodiments 4 and 5 (host:guest = 1:2); and

Fig. 3 is a diagram to show the IR spectrum of 4,5-dichloro-2-n-octyl-3(2H) isothiazolon as the guest compound.

**Preferred Embodiments of the Invention:**

The present invention will be described below in further details with referring to preferred embodiments. It is obvious, however, that the present invention is not limited to these embodiments.

**Embodiment 1:**

Into 10 ml of methanol, 1.4g of 4,5-dichloro-2-n-octyl-3(2H) isothiazolon (from Rohm and Haas) is solved and 1 g of 1,1,2,2-tetrakis (4-hydroxy phenyl) ethane is added. The liquid is heated and stirred until complete solution. After the liquid becomes transparent, it is further stirred for five minutes at 60°C. The liquid is left still at the room temperature for 24 hours and then the deposition is suctioned and filtered. The solid thus obtained is dried in vacuum at the room temperature to have light yellow powder of clathrate compound with 4,5-dichloro-2-n-octyl-3(2H) isothiazolon and 1, 1, 2, 2-tetrakis (4-hydroxy phenyl) ethane at the ratio of 1:1 (mole ratio).

The clathrate compound is checked by TG-DTA analysis and IR analysis.

Fig. 1 shows the IR spectrum of the obtained clathrate compound (KBr method) and Fig. 3 shows the IR spectrum of 4,5-dichloro-2-n-octyl-3(2H) isothiazolon as the guest compound (KBr method).

**Embodiment 2:**

In warm water bath at 60°C, 0.9g of 4,5-dichloro-2-n-octyl-3(2H) isothiazolon is molten. 1.2 g of 1,1,2,2-tetrakis (4-hydroxy phenyl) ethane is added to it and the mixture is stirred for 10 minutes so as to cause reaction. Reaction proceeds quickly and results in white powder of clathrate compound with 4,5-dichloro-2-n-octyl-3(2H) isothiazolon and 1,1,2,2 tetrakis (4-hydroxy phenyl) ethane at the ratio of 1:1 (mole ratio).

The clathrate compound is checked by TG-DTA analysis and IR analysis. It is found that the same substance as in Embodiment 1 is generated.

**Embodiment 3:**

1.2 g of light brown 4,5-dichloro-2-n-octyl-3(2H) isothiazolon powder (from Rohm and Haas) and 0.9 g of white 1,1,2,2-tetrakis (4-hydroxy phenyl) ethane powder are put into a mortar. At the room temperature, the mixture is ground with a pestle for 10 minutes of reaction. Reaction proceeds quickly and results in white powder clathrate compound with 4,5-dichloro-2-n-octyl-3(2H) isothiazolon and 1,1,2,2 tetrakis (4-hydroxy phenyl) ethane at the ratio of 1:1 (mole ratio).

The clathrate compound is checked by TG-DTA analysis and IR analysis. It is found that the same substance as in Embodiment 1 is generated.

**Embodiment 4:**

Into 10 ml of methanol, 2.8 g of 4,5-dichloro-2-n-octyl-3(2H) isothiazolon is solved and 1 g of 1,1,2,2-tetrakis (4-hydroxy phenyl) ethane is added. The mixture is heated and stirred until complete solution. After the liquid becomes transparent, it is further stirred for 5 minutes at 60°C. The liquid is left still at the room temperature for 48 hours and then the deposition is suctioned and filtered. The solid thus obtained is dried in vacuum at the room temperature to have light yellow crystallized clathrate compound with 4,5-dichloro-2-n-octyl-3(2H) isothiazolon and 1,1,2,2 tetrakis (4-hydroxy phenyl) ethane at the ratio of 2:1 (mole ratio).

The clathrate compound is checked by TG-DTA analysis and IR analysis. Fig. 2 shows the IR spectrum of the clathrate compound obtained.

**Embodiment 5:**

In warm water bath at 60°C, 1.8 g of 4,5-dichloro-2-n-octyl-3(2H) isothiazolon is molten. 1.2 g of 1,1,2,2-tetrakis (4-hydroxy phenyl) ethane is added to it and the mixture is stirred for 30 minutes so as to cause reaction. Reaction proceeds quickly and results in light yellow crystallized clathrate compound with 4,5-dichloro-2-n-octyl-3(2H) isothiazo-

Ion and 1,1,2,2 tetrakis (4-hydroxy phenyl) ethane at the ratio of 2:1 (mole ratio).

The clathrate compound is checked by TG-DTA analysis and IR analysis. It is found that the same substance as in Embodiment 4 is generated.

Table 1 shows conditions for clathrate compound preparation, guest/host mole ratio of the obtained clathrate compound and the temperature to release the guest organic compound.

Table I

| Embodiment | Reaction condition | | | Guest/host mole ratio | Guest release temperature (°C) |
|---|---|---|---|---|---|
| | Solvent | Temperature(°C) | Time(Min.) | | |
| 1 | Methanol | 60 | 5 | 1.0 | 195 |
| 2 | Not in use | 60 | 10 | 1.0 | 193 |
| 3 | Not in use | Room temperature | 10 | 1.0 | 197 |
| 4 | Methanol | 60 | 5 | 2.0 | 204 |
| 5 | Not in use | 60 | 10 | 2.0 | 205 |

**Embodiment 6:**

Ship bottom stain-proofing paint including the clathrate compound according to Embodiment 4 is prepared according to the recipe shown in Table 2. Mixture is made by blender for 20 minutes.

**Reference 1:**

Ship bottom stain-proofing paint containing 4,5-dichloro-2-n-octyl-3(2H) isothiazolon not made into clathrate by tetrakis phenol is prepared according to the recipe in Table 2. Mixture is made in the same way as Embodiment 6.

Table 2

| Paint composition | Embodiment 6 | Reference 1 | No addition |
|---|---|---|---|
| Styrene-butadiene rubber | 4 | 4 | 4 |
| Rosin | 8 | 8 | 8 |
| Triclecyl phosphate | 2 | 2 | 2 |
| Copper suboxide (Cu2O) | 40 | 40 | 40 |
| Ferric Oxide | 10 | 10 | 10 |
| Clathrate obtained in Embodiment 4 (58.6% AI) | 5.1 | 0 | 0 |
| Sea Nine 211 (30% AI) *1 | 0 | 10 | 0 |
| Benton 38 *2 | 1 | 1 | 1 |
| Disparon 4200-20 *3 | 1 | 1 | 1 |
| Xylene | 28.9 | 24 | 34 |
| Total | 100 | 100 | 100 |

(Values represent part by weight.)

*1: Product of Rohm and Haas containing 4,5-dichloro-2-n-octyl-3(2H) isothiazolon for 30% and xylene for 70%

*2 Product of NL Chemical containing including hectolite clay for 20% and quaternary amine for 38%

*3 Product of Kusumoto Chemicals Ltd. containing polyethylene oxide for 20% and xylene for 80%

**Industrial Field:**

Test Example 1:

The stain-proofing paints prepared according to Embodiment 6 and Reference 1 are applied to FRP panels (7 x 14 $cm^2$, 2 mm thick) and dried. The panels are placed about 1 m below the sea surface in Kiitabe Gulf, Wakayama Prefecture to check their stain-proofing performance against sea creatures for six months.

For evaluation, the area occupied by sea creatures is visually checked against the criteria shown in Table 3. Table 4 shows the test results.

Table 3

| Score | Percentage of Area with Clinging |
|---|---|
| 1 | No clinging |
| 2 | Less than 1% |
| 3 | Less than 5% |
| 4 | 5 to 10% |
| 5 | 10 to 20% |
| 6 | 20 to 50% |
| 7 | 50% or more |

Table 4

| Example \ Term (months) | 1 | 2 | 3 | 4 | 5 | 6 |
|---|---|---|---|---|---|---|
| Embodiment 6 | 1 | 1 | 1 | 1 | 1 | 1 |
| Reference 1 | 1 | 1 | 1 | 1 | 2 | 2 |
| No additive | 1 | 3 | 7 | 7 | 7 | 7 |

Test Example 2 - Solution from ship bottom stain-proofing paint film

Each of the stain-proofing paints prepared according to Embodiment 6 and Reference 1 is applied twice to a part on both sides of an FRP panel (Dried film thickness: about 60 μm x 2). The panel is dried at the room temperature for three days and then dipped in artificial sea water (60 liters) in a tank (water temperature: 21 to 26° C). 4,5-dichloro-2-n-octyl-3(2H) isothiazolon dissolved from the paint film is measured once a week by bubbling method to determine the dissolution ratio. Table 5 shows the results.

Bubbling method:

To a beaker containing 100 ml of artificial sea water (pH: 8.2), an air bubbler (air blow amount: approx. 200 ml/min.) is set and the water temperature is kept constant at 25 ±1 °C. After dipped in the above tank for a certain period, the FRP test plate is taken out and kept in the beaker for one hour. After that, the FRP test plate is returned to the original tank and a certain amount in the beaker is sampled for measurement of effective ingredient concentration

with HPLC.

$$\text{Solution ratio } (\mu g/cm^2/day) = X \times Y \times T / Z$$

where

X is the effective concentration in test solution (μg/ml),
Y is the amount of sea water in the bath (ml),
T is the time (24 hours=one day), and
Z is the paint film area ($cm^2$).

Table 5

| Embodiment \ No. of days | 1 | 7 | 14 | 21 | 28 |
|---|---|---|---|---|---|
| Embodiment 6 | 22.3 | 18.9 | 15.6 | 13.3 | 10.0 |
| Reference 1 | 137 | 31.9 | 22.3 | 17.5 | 15.3 |

Numerics indicate dissolution ratios (μ g/$cm^2$/day)

The present invention relates to a new clathrate compound having a tetrakisphenol as the host compound and 4,5-dichloro-2-alkyl-3(2H) isothiazolon as the guest compound. The new clathrate compound of the present invention has characteristics below.

1) It is quite easy to prepare a new clathrate compound having a tetrakis phenols as the host compound and 4,5-dichloro-2-alkyl-3(2H) isothiazolon as the guest compound.
2) The clathrate compound of the present invention is quite safely and easily handled since, even when 4,5-dichloro-2-alkyl-3(2H) isothiazolon with irritating property to human bodies are contained as the guest, the irritation is felt little.
3) 4,5-dichloro-2-alkyl-3(2H) isothiazolon is released from the clathrate compound at a higher temperature. (For example, in case where 4,5-dichloro-2-n-octyl-3(2H) isothiazolon is the guest compound, while the melting point of the guest compound is about 40 °C, re-release temperature from the clathrate compound is 190 °C or more). This enables heat treatment of the clathrate compound.
4) When used as the stain-proofing agent, it has moderate releasing property which leads to long duration of effect.

## Claims

1. A clathrate compound comprising a tetrakisphenol expressed by formula (I) as the host compound and 4,5-dichloro-2-alkyl-3(2H)-isothiazolone as the guest compound.

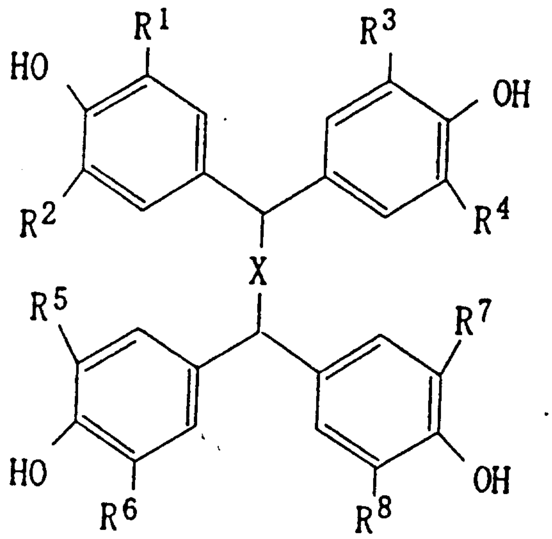

(I)

(In the formula, X represents $(CH_2)n$ where n may be 0, 1, 2 or 3; and each of R1 to R8 represents a substituent selected from the group consisting of a hydrogen atom, a $C_1$-$C_3$ alkyl group, a halogen atom, a $C_1$-$C_3$ alkoxy group and a substitutable phenyl group).

2. A preparation method of the clathrate compound of claim 1, wherein the compound is obtained through reaction between a tetrakisphenol expressed by formula (I) and 4,5-dichloro-2-alkyl-3(2H)-isothiazolone.

3. A stain-proofing agent characterized in that it contains as effective ingredients one or a plurality of clathrate compounds comprising the tetrakisphenol expressed by formula (I) as the host compound and 4,5-dichloro-2-alkyl-3(2H)-isothiazolone as the guest compound.

**Patentansprüche**

1. Clathratverbindung, umfassend ein Tetrakisphenol, angegeben durch die Formel (I), als Wirtsverbindung und 4,5-Dichlor-2-alkyl-3(2H)-isothiazolon als Gastverbindung

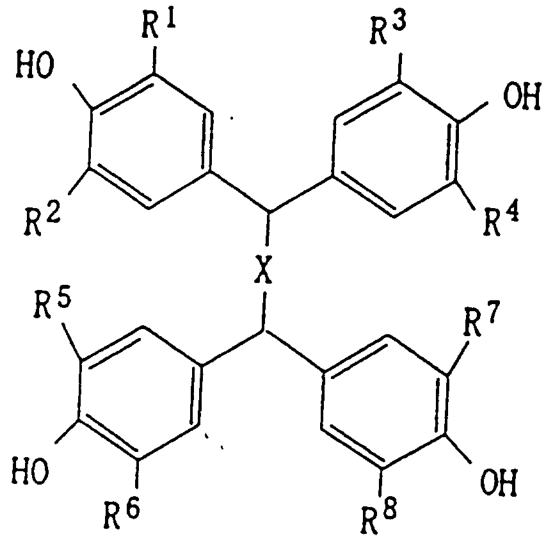

(I)

(in der Formel bedeutet X $(CH_2)n$, wobei n 0, 1, 2 oder 3 sein kann und jedes $R^1$ bis $R^8$ bedeutet einen Substituenten, ausgewählt aus der Gruppe bestehend aus einem Wasserstoffatom, einer $C_1$-$C_3$-Alkylgruppe, einem Halogenatom, einer $C_1$-$C_3$-Alkoxygruppe und einer substituierbaren Phenylgruppe).

2. Verfahren zur Herstellung der Clathratverbindung nach Anspruch 1, wobei die Verbindung erhalten wird durch Reaktion zwischen einem Tetrakisphenol, angegeben durch die Formel (I) und 4,5-Dichlor-2-alkyl-3(2H)-isothiazolon.

3. Mittel zur Verhinderung von Verfärbungen und/oder Schimmelbildung, dadurch gekennzeichnet, daß es als wirksame Bestandteile eine oder eine Mehrzahl von Clathratverbindungen, umfassend das Tetrakisphenol, angegeben durch die Formel (I), als Wirtsverbindung und 4,5-Dichlor-2-alkyl-3(2H)-isothiazolon als Gastverbindung, enthält.

**Revendications**

1. Composé clathrate comprenant un tétrakisphénol exprimé par la formule (I) en tant que composé hôte et de la 4,5-dichloro-2-alkyl-3(2H)-isothiazolone en tant que composé invité

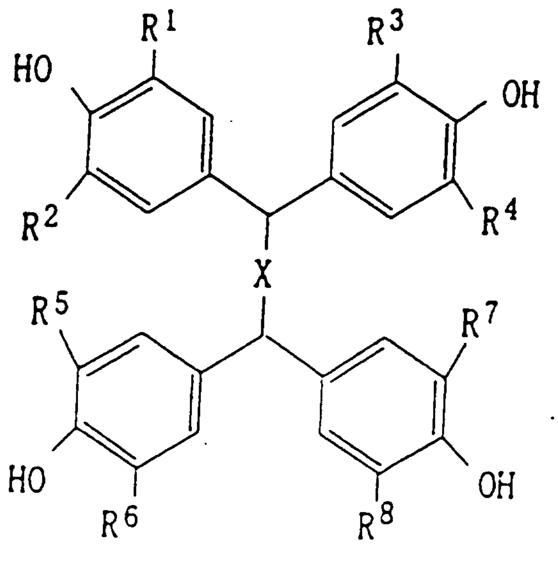

(I)

(dans la formule, X représente $(CH_2)_n$ où n peut être 0, 1, 2 ou 3; et chacun de R1 à R8 représente un substituant sélectionné dans le groupe consistant en un atome d'hydrogène, un groupe alkyle $C_1$-$C_3$, un atome d'halogène, un groupe alcoxy $C_1$-$C_3$ et un groupe phényle substituable.

**2.** Méthode de préparation d'un composé clathrate de la revendication 1, où le composé est obtenu par réaction entre un tétrakisphénol exprimé par la formule (I) et de la 4,5-dichloro-2-alkyl-3(2H)-isothiazolone.

**3.** Agent antisalissure caractérisé en ce qu'il contient, comme ingrédients efficaces, un ou un certain nombre de composés clathrate comprenant le tétrakisphénol exprime par la formule (I) comme composé hôte et la 4,5-dichloro-2-alkyl-3(2H)-isothiazolone comme composé invité.

Fig. 1

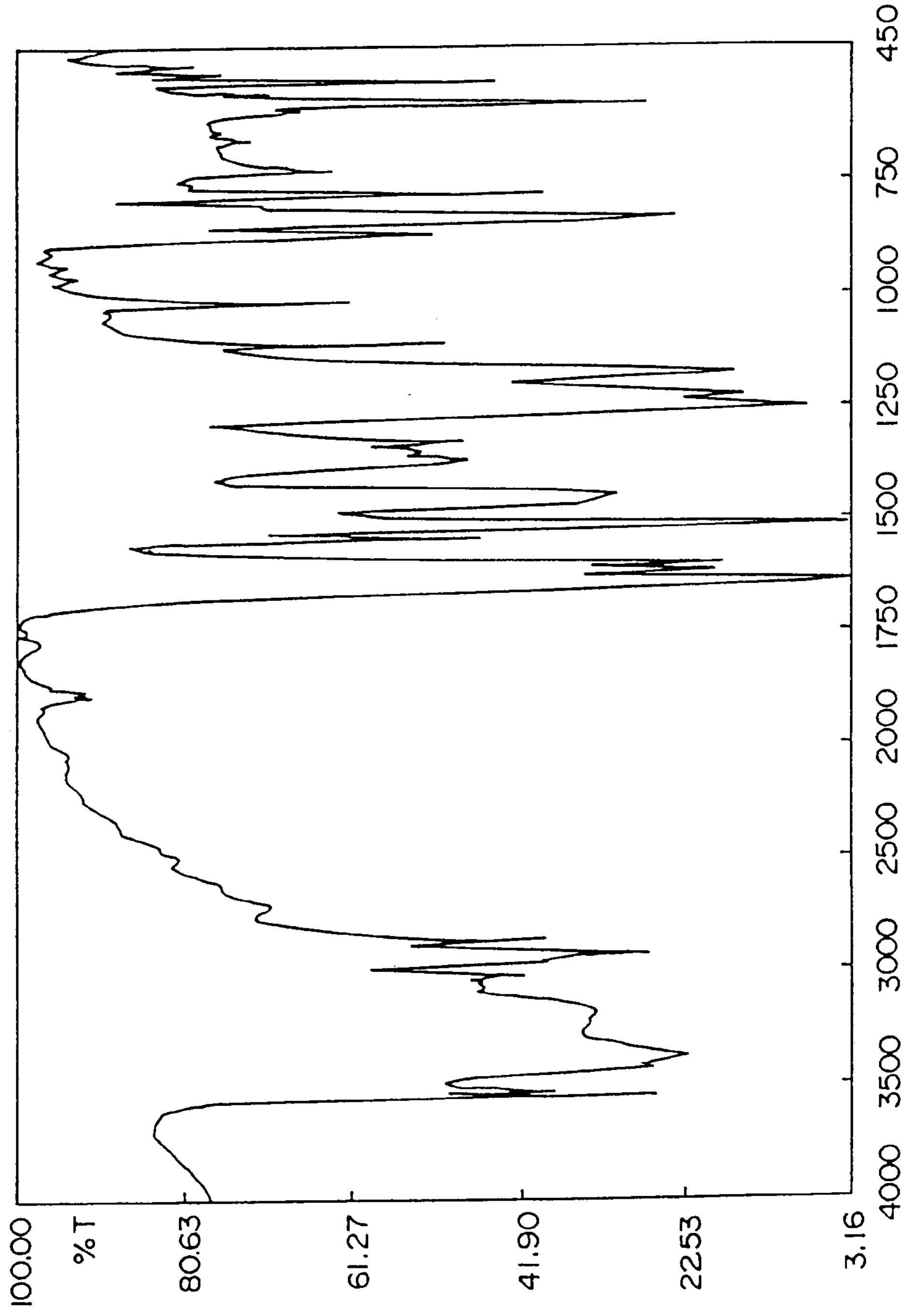
%T
100.00
80.63
61.27
41.90
22.53
3.16
4000
3500
3000
2500
2000
1750
1500
1250
1000
750
450
cm-1

Fig. 2

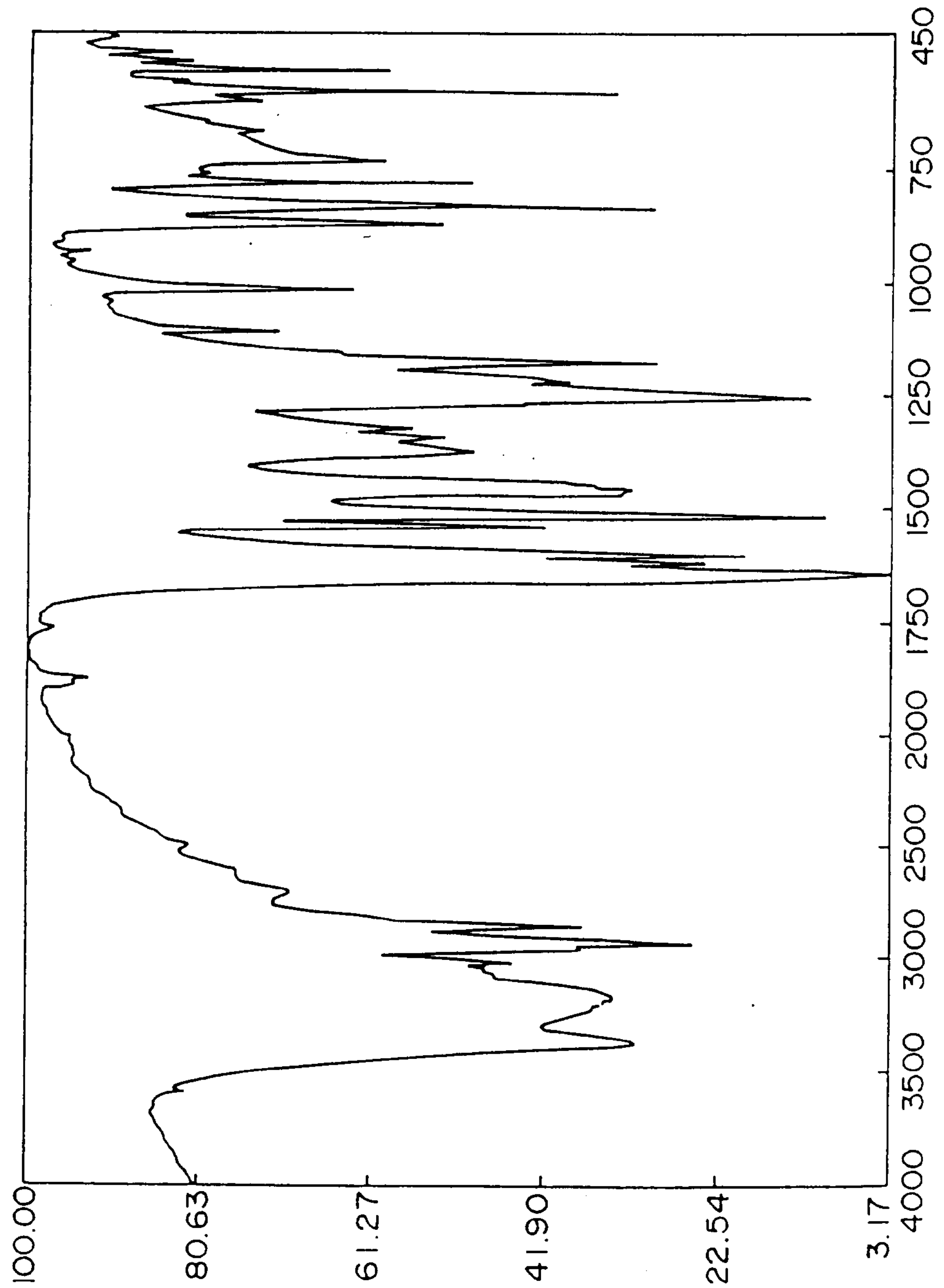
100.00
80.63
61.27
41.90
22.54
3.17
4000
3500
3000
2500
2000
1750
1500
1250
1000
750
450
cm⁻¹

Fig. 3

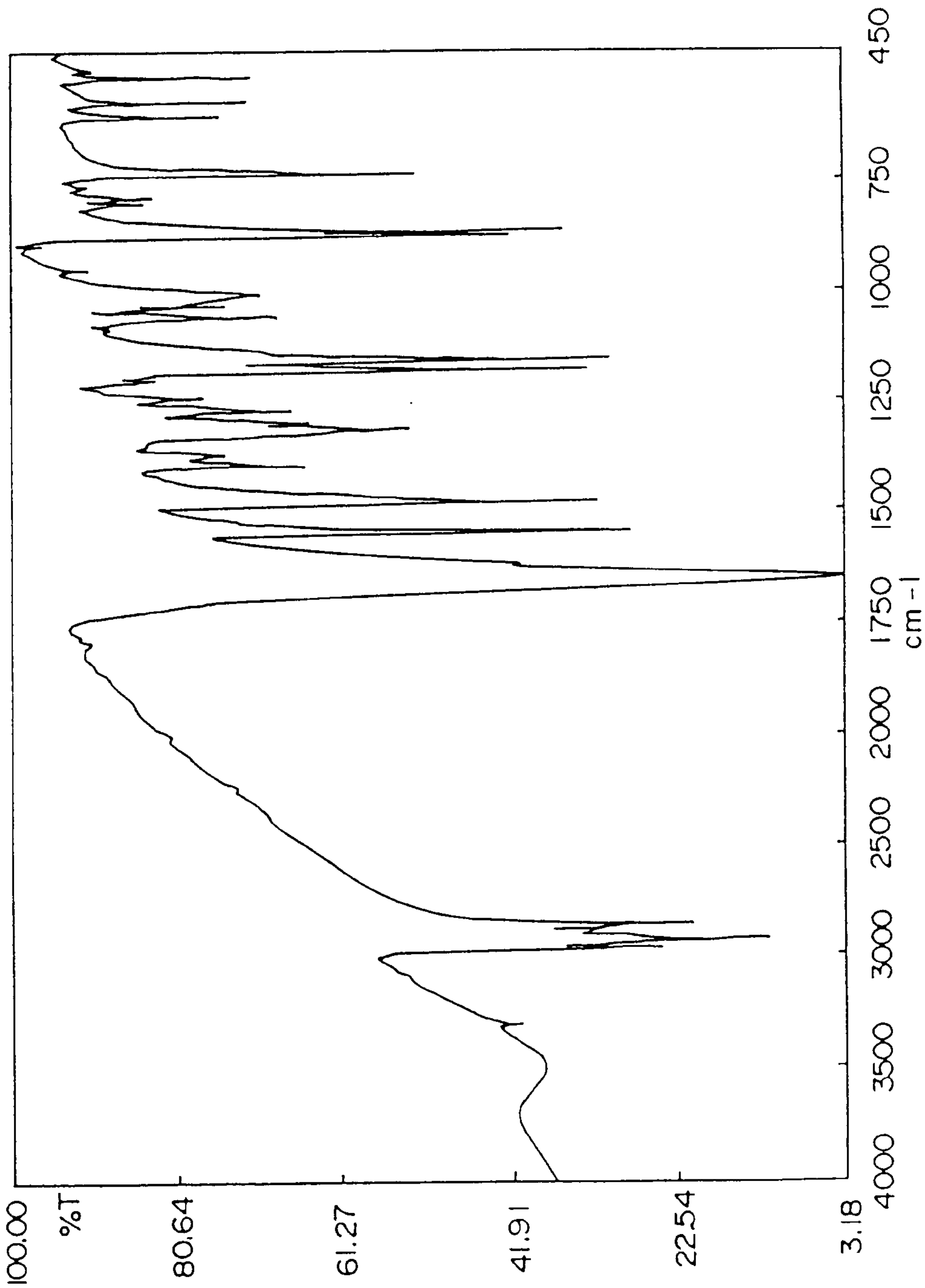
%T
100.00
80.64
61.27
41.91
22.54
3.18
4000
3500
3000
2500
2000
1750
1500
1250
1000
750
450
cm-1